# EUROPÄISCHE PATENTSCHRIFT

(11) **EP 0 739 408 B1**
(45) Veröffentlichungstag und Bekanntmachung des Hinweises auf die Patenterteilung: **28.07.1999**
(21) Anmeldenummer: 95905114.5
(22) Anmeldetag: 03.01.1995
(51) Int. Cl.: C11D 1/645, B03D 1/01, A61K 7/50

(54) **TENSIDGEMISCHE**
TENSIDE MIXTURES
MELANGES TENSIOACTIFS

(30) Priorität: 12.01.1994 DE 4400632
(43) Veröffentlichungstag der Anmeldung: 30.10.1996
(73) Patentinhaber: Henkel Kommanditgesellschaft auf Aktien, 40191 Düsseldorf (DE)
(72) Erfinder: FABRY, Bernd, D-41352 Korschenbroich (DE)
(86) Internationale Anmeldenummer: EP9500008
(87) Internationale Veröffentlichungsnummer: WO9519415

(56) Entgegenhaltungen:
- EP-A- 0 285 768
- US-A- 2 703 798
- US-A- 3 424 680

## Beschreibung

### Gebiet der Erfindung

Die Erfindung betrifft Tensidgemische mit verbesserten oberflächenaktiven Eigenschaften, enthaltend kurzkettige und langkettige Fettsäure-N-alkylpolyhydroxyalkylamide in ausgewählten synergistischen Mischungsverhältnissen.

### Stand der Technik

Fettsäure-N-alkylpolyhydroxyalkylamide stellen nichtionische Tenside dar, die üblicherweise durch reduktive Aminierung eines reduzierenden Zuckers mit Ammoniak, einem Alkylamin oder einem Alkanolamin und nachfolgende Acylierung mit einer Fettsäure, einem Fettsäurealkylester oder einem Fettsäurechlorid erhalten werden können. Hinsichtlich der Verfahren zu ihrer Herstellung sei auf die US-Patentschriften **US 1,985,424, US 2,016,962** und **US 2,703,798** sowie die Intemationale Patentanmeldung **WO 92/06984** verwiesen. Eine Übersicht zu diesem Thema von H.Kelkenberg findet sich in **Tens.Surf.Det. 25, 8 (1988)**. Vorzugsweise leiten sich die Fettsäure-N-alkylpolyhydroxyalkylamide von reduzierenden Zuckern mit 5 oder 6 Kohlenstoffatomen, insbesondere von der Glucose ab. Die bevorzugten Fettsäure-N-alkylpolyhydroxyalkylamide stellen daher Fettsäure-N-alkylglucamide dar. Fettsäure-N-alkylglucamide werden seit einiger Zeit als milde nichtionische Tenside mit guten Schaum- und Reinigungsvermögen in Handgeschirrspülmitteln eingesetzt. Die Verwendung dieser Stoffe ist dabei Gegenstand einer Vielzahl von Veröffentlichungen. Aus der Europäischen Patentanmeldung **EP-A1 0285768** (Hüls) ist beispielsweise ihr Einsatz als Verdickungsmittel bekannt. In der Französischen Offenlegungsschrift **FR-A 1580491** (Henkel) werden wäßrige Detergensgemische auf Basis von Sulfaten und/oder Sulfonaten, Niotensiden und gegebenfalls Seifen beschrieben, die Fettsäure-N-alkylglucamide als Schaumregulatoren enthalten.

Gegenstand der Intemationalen Patentanmeldungen **WO 92/06153, WO 92/06156, WO 92/06157, WO 92/06158, WO 92/06159** und **WO 92/06160** (Procter & Gamble) sind Mischungen von Fettsäure-N-alkylglucamiden mit anionischen Tensiden, Tensiden mit Sulfat- und/oder Sulfonatstruktur, Ethercarbonsäuren, Ethersulfaten, Methylestersulfonaten und nichtionischen Tensiden. Die Verwendung dieser Stoffe in den unterschiedlichsten Wasch-, Spül- und Reinigungsmitteln wird in den Internationalen Patentanmeldungen **WO 92/06152, WO 92/06154, WO 92/06155, WO 92/06161, WO 92/06162, WO 92/06164, WO 92/06170, WO 92/06171** und **WO 92/06172** (Procter & Gamble) beschrieben. Trotz dieses umfangreichen Stands der Technik besteht nach wie vor ein Bedürfnis nach Fettsäure-N-alkylpolyhhydroxyalkylamiden mit verbesserten Eigenschaften, insbesondere einem stärkeren Schaumvermögen auch in hartem Wasser, besseren Netzzeiten, gesteigerter Waschkraft und höherer dermatologischer Verträglichkeit.

Die Aufgabe der vorliegenden Erfindung hat somit darin bestanden, Fettsäure-N-alkylpolyhydroxyalkylamide zur Verfügung zu stellen, die das geforderte komplexe Eigenschaftsprofil erfüllen.

### Beschreibung der Erfindung

Gegenstand der Erfindung sind Tensidgemische mit verbesserten oberflächenaktiven Eigenschaften, enthaltend
(a) Fettsäure-N-alkylpolyhydroxyalkylamide der Formel **(I)**, 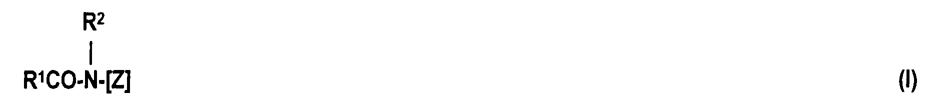 in der R¹CO für einen aliphatischen Acylrest mit 6 bis 10 Kohlenstoffatomen, R² für einen Alkyl- oder Hydroxyalkylrest mit 1 bis 4 Kohlenstoffatomen und [Z] für einen linearen oder verzweigten Polyhydroxyalkylrest mit 3 bis 12 Kohlenstoffatomen und 3 bis 10 Hydroxylgruppen steht, und
(b) Fettsäure-N-alkylpolyhydroxyalkylamide der Formel **(II)**, 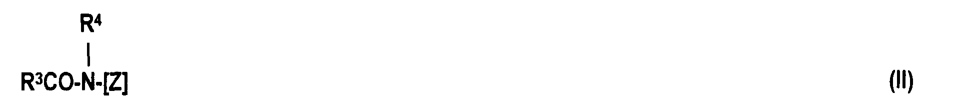 in der R³CO für einen aliphatischen Acylrest mit 12 bis 22 Kohlenstoffatomen, R⁴ für einen Alkyl- oder Hydroxyalkylrest mit 1 bis 4 Kohlenstoffatomen und [Z] für einen linearen oder verzweigten Polyhydroxyalkylrest mit 3 bis 12 Kohlenstoffatomen und 3 bis 10 Hydroxylgruppen steht,
wobei das Gewichtsverhältnis zwischen den Komponenten (a) und b) 80 : 20 bis 20 : 80 beträgt. Überraschenderweise wurde gefunden, daß sich kurzkettige und längerkettige Fettsäure-N-alkylpolyhydroxyalkylamide, insbesondere Fettsäure-N-alkylglucamide hinsichtlich ihres Schaum-, Netz- und Waschvermögens sowie ihrer dermatologischen Verträglichkeit innerhalb bestimmter Mischungsbereiche in synergistischer Weise ergänzen.

### Fettsäure-N-alkylpolyhydroxyalkylamide

Wie bereits ausgeführt, handelt es sich bei den Fettsäure-N- alkylpolyhydroxyalkylamiden um bekannte nichtionische Tenside, die nach einschlägigen bereits genannten Verfahren des Stands der Technik erhalten werden können. Vorzugsweise leiten sich die Fettsäure-N-alkylpolyhydroxyalkylamide von reduzierenden Zuckem mit 5 oder 6 Kohlenstoffatomen, insbesondere von der Glucose ab. Die bevorzugten Fettsäure-N-alkylpolyhydroxyalkylamide stellen daher **Fettsäure-N-alkylglucamide** dar, wie sie durch die Formel **(III)** wiedergegeben werden, 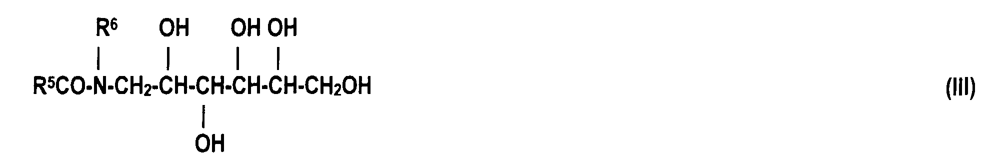 in der R⁵CO und R⁶ die Bedeutungen entsprechend den Formeln **(I)** bzw. **(II)** haben. Weiterhin können sich die Polyhydroxyalkylamide auch von Maltose und Palatinose ableiten. Vorzugsweise entsprechen die Stoffe der Komponente (a) Fettsäure-N-alkylglucamiden der Formel **(I)**, in der R¹CO für einen gesättigten Acylrest mit 8 bis 10 Kohlenstoffatomen und R² für einen Methylrest steht. Typische Beispiele sind Fettsäure-N-methylglucamide, die sich von Caprylsäure, 2-Ethylhexansäure und/oder Caprinsäure sowie deren technischen Gemischen ableiten. Besonders bevorzugt ist der Einsatz eines Methylglucamids auf Basis einer technischen C₈-C₁₀-Fettsäure, die beispielsweise als Vorlauf bei der Destillation von Kokosfettsäure anfällt. Vorzugsweise entsprechen die Stoffe der Komponente (b) Fettsäure-N-alkylglucamiden der Formel **(II)**, in der R³CO für einen gesättigten Acylrest mit 12 bis 18 und insbesondere 12 bis 14 Kohlenstoffatomen und R⁴ für einen Methylrest steht. Typische Beispiele sind Fettsäure-N-alkylglucamide, die sich von Laurinsäure, Myristinsäure, Palmitinsäure, Palmoleinsäure, Stearinsäure, Isostearinsäure, Ölsäure, Elaidinsäure, Petroselinsäure, Linolsäure, Linolensäure sowie deren technischen Gemischen ableiten. Besonders bevorzugt ist der Einsatz eines Methylglucamins auf Basis einer technischen C₁₂- C₁₄-Kokosfettsäurefraktion. Die Komponenten (a) und (b) können in den Tensidgemischen in den Gewichtsverhältnissen 80 : 20 bis 20 : 80, vorzugsweise 75 : 25 bis 25 : 75 enthalten sein. Besonders bevorzugt ist ein Verhältnis (a) : (b) von 20 : 80 bis 50 : 50 und insbesondere 25 : 75 bis 40 : 60. Hierbei kommt zum Ausdruck, daß die stärksten Synergien dann erreicht werden, wenn man den längerkettigen Fettsäure-N-alkylglucamiden 20 bis 50 und vorzugsweise 25 bis 40 Gew.-% kürzerkettige Homologe zumischt.

### Weitere Tenside

Die erfindungsgemäßen Tensidgemische können weitere anionische, nichtionische, kationische und/oder amphotere bzw. zitterionische Tenside enthalten, wobei der Anteil der Komponenten (a) und (b) an diesen mindestens temären Gemischen vorzugsweise in Summe 25 bis 75 und insbesondere 40 bis 60 Gew.-% - bezogen auf die Mischungen - beträgt. Typische Beispiele für **anionische Tenside** sind Alkylbenzolsulfonate, Alkansulfonate, Olefinsulfonate, Alkylethersulfonate, Glycerinethersulfonate, α-Methylestersulfonate, Sulfofettsäuren, Alkylsulfate, Fettalkoholethersulfate, Glycerinethersulfate, Hydroxymischethersulfate, Monoglyceridsulfate, Fettsäureamid(ether)sulfate, Sulfosuccinate, Sulfotriglyceride, Amidseifen, Ethercarbonsäuren, Fettsäureisethionate, Sarcosinate, Tauride, Alkyloligoglucosidsulfate, Alkyl(ether)phosphate, Acyllactylate und pflanzliche oder tierischen Eiweißhydrolysate bzw. deren Kondensationsprodukten mit Fettsäuren. Sofem die anionischen Tenside Polyglycoletherketten enthalten, können sie eine konventionelle, vorzugsweise jedoch eine eingeengte Homologenverteilung aufweisen. Typische Beispiele für **nichtionische Tenside** sind Fettalkoholpolyglycolether, Alkylphenolpolyglycolether, Fettsäurepolyglycolester, Fettsäureamidpolygylcolether, Fettaminpolyglycolether, alkoxylierte Triglyceride, Alk(en)yloligoglykoside, Polyolfettsäureester, Zuckerester, Sorbitanester und Polysorbate. Sofern die nichtionischen Tenside Polyglycoletherketten enthalten, können sie eine konventionelle, vorzugsweise jedoch eine eingeengte Homologenverteilung aufweisen. Typische Beispiele für **kationische Tenside** sind quartäre Ammoniumverbindungen und quatemierte Fettsäuretrialkanolaminester. Typische Beispiele für **amphotere bzw. zwitterionische Tenside** sind Alkylbetaine, Alkylamidobetaine, Aminopropionate, Aminoglycinate, Imidazoliniumbetaine und Sulfobetaine. Bei den genannten Tensiden handelt es sich ausschließlich um bekannte Verbindungen. Hinsichtlich Struktur und Herstellung dieser Stoffe sei auf einschlägige Übersichtsarbeiten beispielsweise **J.Falbe (ed.), "Surfactants in Consumer Products", Springer Verlag, Berlin, 1987, S.54-124** oder **J.Falbe (ed.), "Katalysatoren, Tenside und Mineralöladditive", Thieme Verlag, Stuttgart, 1978, S.123-217** verwiesen.

Die Herstellung der wäßrigen Detergensgemische kann durch einfaches mechanisches Vermischen der wäßrigen Lösungen der Komponenten, gegebenenfalls bei erhöhten Temperaturen von 30 bis 50°C erfolgen; eine chemische Reaktion findet hierbei nicht statt.

### Oberflächenaktive Mittel

Im folgenden werden weitere Gegenstände beschrieben, auf die sich die Erfindung ebenfalls erstreckt:
- **Pulverförmige Universalwaschmittel,** enthaltend 10 bis 30 Gew.-% - bezogen auf das Mittel - der erfindungsgemäßen Tensidgemische sowie gegebenenfalls weitere Hilfs- und Zusatzstoffe.
- **Flüssige Universalwaschmittel,** enthaltend 10 bis 70 Gew.-% - bezogen auf das Mittel - der erfindungsgemäßen Tensidgemische sowie gegebenenfalls weitere Hilfs- und Zusatzstoffe.
- **Flüssige Feinwaschmittel,** enthaltend 10 bis 50 Gew.-% - bezogen auf das Mittel - der erfindungsgemäßen Tensidgemische sowie gegebenenfalls weitere Hilfs- und Zusatzstoffe.
- **Flüssige Reinigungs- und Desinfektionsmittel,** enthaltend 10 bis 30 Gew.-% - bezogen auf das Mittel - der erfindungsggemäßen Tensidgemische sowie gegebenenfalls weitere Hilfs- und Zusatzstoffe.
- **Handgeschirrspülmittel**, enthaltend 5 bis 25 Gew.-% - bezogen auf das Mittel - der erfindungsgemäßen Tensidgemische sowie gegebenenfalls weitere Hilfs- und Zusatzstoffe. In diesem Zusammenhang besonders bevorzugt sind Mittel, die 5 bis 10 Gew.-% der erfindungsgemäßen Tensidgemische, 5 bis 10 Gew.-% Fettalkoholethersulfate, 1 bis 5 Gew.-% Alkylbetaine und/oder 1 bis 5 Gew.-% Alkyloligoglucoside enthalten.
- **Maschinengeschirrspülmittel**, enthaltend 5 bis 25 Gew.-% - bezogen auf das Mittel - der erfindungsgemäßen Tensidgemische sowie gegebenenfalls weitere Hilfs- und Zusatzstoffe.
- **Klarspülmittel,** enthaltend 10 bis 50 Gew.-% - bezogen auf die Mittel - der erfindungsgemäßen Tensidgemische sowie gegebenenfalls weitere Hilfs- und Zusatzstoffe.
- **Wäscheweichspülmittel,** enthaltend 2 bis 35 Gew.-% - bezogen auf die Mittel - der erfindungsgemäßen Tensidgemische sowie gegebenenfalls weitere Hilfs- und Zusatzstoffe.
- **Haarshampoos**, enthaltend 10 bis 30 Gew.-% - bezogen auf das Mittel - der erfindungsgemäßen Tensidgemische sowie gegebenenfalls weitere Hilfs- und Zusatzstoffe.
- **Haarspülungen**, enthaltend 10 bis 30 Gew.-% - bezogen auf das Mittel - der erfindungsgemäßen Tensidgemische sowie gegebenenfalls weitere Hilfs- und Zusatzstoffe.
- **Schaumbäder**, enthaltend 10 bis 30 Gew.-% - bezogen auf das Mittel - der erfindungsgemäßen Tensidgemische sowie gegebenenfalls weitere Hilfs- und Zusatzstoffe.
- **Syndetseifen**, enthaltend 10 bis 50 Gew.-% - bezogen auf das Mittel - der erfindungsgemäßen Tensidgemische sowie gegebenenfalls weitere Hilfs- und Zusatzstoffe.
- **Sammler für die Flotation nichtsulfidischer Erze**, enthaltend 10 bis 95 Gew.-% - bezogen auf die Mittel - der erfindungsgemäßen Tensidgemische sowie gegebenenfalls weitere Hilfs- und Zusatzstoffe.

### Wasch- und Reinigungsmittel

Wasch- und Reinigungsmittel auf Basis der erfindungsgemäßen Detergensgemische können als Hilfs- und Zusatzstoffe beispielsweise Builder, Salze, Bleichmittel, Bleichaktivatoren, optische Aufheller, Vergrauungsinhibitoren, Soil-repellants, Lösungsvermittler, Entschäumer und Enzyme enthalten. Übliche **Builder** sind Natriumaluminiumsilicate (Zeolithe), Phosphate, Phosphonate, Ethylendieamintetraessigsäure, Nitrilotriacetat, Citronensäure und/oder Polycarboxylate. Als **Salze** bzw. Stellmittel kommen beispielsweise Natriumsulfat, Natriumcarbonat oder Natriumsilicat (Wasserglas) in Betracht. Als typische Einzelbeispiele für weitere Zusatzstoffe sind Natriumborat, Stärke, Saccharose, Polydextrose, TAED, Stilbenverbindungen, Methylcellulose, Toluolsulfonat, Cumolsulfonat, langkettige Seifen, Silicone, Mischether, Lipasen und Proteasen zu nennen. Typische **soil-repellants** stellen Polymere dar, die vorzugsweise Ethylenterephthalat- und/oder Polyethylenglycolterephthalatgruppen enthalten, wobei das Molverhältnis Ethylenterephthalat zu Polyethylenglycolterephthalat im Bereich von 50 : 50 bis 90 : 10 liegen kann. Das Molekulargewicht der verknüpfenden Polyethylenglycoleinheiten liegt vorzugsweise im Bereich von 750 bis 5000, d.h., der Ethoxylierungsgrad der Polyethylenglycolgruppenhaltigen Polymere kann ca. 15 bis 100 betragen. Die Polymeren zeichnen sich durch ein durchschnittliches Molekulargewicht von etwa 5000 bis 200.000 aus und können eine Block-, vorzugsweise aber eine Random-Struktur aufweisen. Beispiele für handelsübliche Polymere sind die Produkte Milease® T (ICI) oder Repelotex® SRP 3 (Rhöne-Poulenc).

### Kosmetische Mittel

**Haarshampoos, Haarlotionen** oder **Schaumbäder** auf Basis der erfindungsgemäßen Tensidgemische können als Hilfs- und Zusatzstoffe beispielsweise Emulgatoren, Ölkomponenten, Fette und Wachse, Verdickungsmittel, Überfettungsmittel, biogene Wirkstoffe, Filmbildner, Duftstoffe, Farbstoffe, Perlglanzmittel, Konservierungsmittel und pH-Regulatoren enthalten. Als **Ölkörper** kommen beispielsweise Guerbetalkohole auf Basis von Fettalkoholen mit 6 bis 18, vorzugsweise 8 bis 10 Kohlenstoffatomen, Ester von linearen C₆-C₂₀-Fettsäuren mit linearen C₆-C₂₀-Fettalkoholen, Ester von verzweigten C₆-C₁₃-Carbonsäuren mit linearen C₁₆-C₁₈-Fettalkoholen, Ester von linearen C₁₀-C₁₈-Fettsäuren mit verzweigten Alkoholen, insbesondere 2-Ethylhexanol, Ester von linearen und/oder verzweigten Fettsäuren mit zweiwertigen Alkoholen und/oder Guerbetalkoholen, Triglyceride auf Basis C₆-C₁₀-Fettsäuren, pflanzliche Öle, verzweigte primäre Alkohole, substituierte Cyclohexane und/oder Dialkylether in Betracht. Als **Emulgatoren** kommen sowohl bekannte W/O- als auch O/W- Emulgatoren in Frage. Typische Beispiele für **Fette** sind Glyceride, als **Wachse** kommen u.a. Bienenwachs, Paraffinwachs oder Mikrowachse in Frage. Geeignete **Verdickungsmittel** sind beispielsweise vemetzte Polyacrylsäuren und deren Derivate, Polysaccharide, insbesondere Xanthan-Gum, Guar-Guar, Agar-Agar, Alginate und Tylosen, Carboxymethylcellulose und Hydroxyethylcellulose, ferner Fettalkohole, Monoglyceride und Fettsäuren, Polyacrylate, Polyvinylalkohol und Polyvinylpyrrolidon. Unter **biogenen Wirkstoffen** sind beispielsweise Pflanzenextrakte, Eiweißhydrolysate und Vitaminkomplexe zu verstehen. Gebräuchliche Filmbildner sind beispielsweise Polyvinylpyrrolidon, Vinylpyrrolidon-Vinylacetat-Copolymerisate, Polymere der Acrylsäurereihe, quatemäre Cellulose-Derivate und ähnliche Verbindungen. Als **Konservierungsmittel** eignen sich beispielsweise Formaldehydlösung, p-Hydroxybenzoat oder Sorbinsäure. Als **Perlglanzmittel** kommen beispielsweise Glycoldistearinsäureester wie Ethylenglycoldistearat, aber auch Fettsäuren und Fettsäuremonoglycolester in Betracht. Als **Farbstoffe** können die für kosmetische Zwecke geeigneten und zugelassenen Substanzen verwendet werden, wie sie beispielsweise in der Publikation "**Kosmetische Färbemittel" der Farbstoffkommission der Deutschen Forschungsgemeinschaft, veröffentlicht im Verlag Chemie, Weinheim, 1984**, zusammengestellt sind. Diese Farbstoffe werden üblicherweise in Konzentrationen von 0,001 bis 0,1 Gew.-%, bezogen auf die gesamte Mischung, eingesetzt.

Der Gesamtanteil der Hilfs- und Zusatzstoffe kann 1 bis 50, vorzugsweise 5 bis 40 Gew.-% - bezogen auf die Mittel - betragen. Die Herstellung der Mittel kann in an sich bekannter Weise, d.h. beispielsweise durch Heiß-, Kalt-, Heiß- Heiß/Kalt- bzw. PIT-Emulgierung erfolgen. Hierbei handelt es sich um ein rein mechanisches Verfahren, eine chemische Reaktion findet nicht statt.

### Gewerbliche Anwendbarkeit

Die erfindungsgemäßen Tensidgemische zeichnen sich durch eine synergistische Verstärkung der Wasch-, Spül-, Schaum- und Reinigungsleistung sowie eine vorteilhafte hautkosmetische und ökotoxikologische Verträglichkeit aus. Im Bereich eines Mischungsverhältnisses der Komponenten (a) und (b) von 20 : 80 bis 50 : 50 und vorzugsweise 25 : 75 bis 40 : 60 wurde ein Minimum der Reizsummenscores und damit ein Optimum der dermatologischen Verträglichkeit gefunden. Ein weiterer Gegenstand der Erfindung betrifft daher die Verwendung der erfindungsgemäßen Tensidgemische zur Herstellung von Wasch-, Spül-, Reinigungs-, Avivage- und Flotationshilfsmitteln, Produkten zur Haar- und Körperpflege, in denen sie in Mengen von 1 bis 99, vorzugsweise 10 bis 30 Gew.-% - bezogen auf die Mittel - enthalten sein können.

### Beispiele

- **Schaumvermögen**. Die Beurteilung des Schaumvermögens wurde gemäß **DIN 53 902, Teil 1**, nach der Götte-Schlagschaum-Methode durchgeführt. Es wurden 1 Gew.-%ige Tensidlösungen in Wasser von 16° Härte eingesetzt; die Temperatur betrug 20°C; angegeben wird das Basisschaumvolumen in ml.
- **Netzvermögen**. Die Beurteilung des Netzvermögens wurde gemäß **DIN 53 901** nach der Tauchnetzmethode durchgeführt. Es wurden 1 Gew.-%ige Tensidlösungen in Wasser von 16° Härte eingesetzt; die Temperatur betrug 20°C; angegeben wird die Netzzeit in s.
- **Tellerspülvermögen**. Die Ermittlung des Tellerspülvermögens wurde mit Hilfe des Teller-Testes [**Fette, Seifen, Anstrichmitt., 74, 163 (1972)**] durchgeführt. Hierzu wurden Teller mit einem Durchmesser von 14 cm mit je 2 ml Rindertalg (Säurezahl 9-10) angeschmutzt und 24 h bei Raumtemperatur gelagert. Anschließend wurden die Teller bei 50°C mit 5 l Leitungswasser der Härte 16°d gespült. Die Prüfmischung wurde mit einer Dosierung von 0,15 g Aktivsubstanz/l eingesetzt. Der Spülversuch wurde abgebrochen, sobald der Schaum an der Oberfläche völlig verschwunden war; angegeben wird die Anzahl gespülter Teller.
- **Waschvermögen**. Die Untersuchung des Waschvermögens wurde in einem Launderometer an einer Staub-Hauffett-Anschmutzung auf Polyester-Baumwoll (veredelt) Gewebe bei 40°C durchgeführt. Die Beurteilung der Aufhellung des gewaschenen Gewebes erfolgte durch photometrische Remissionsmessung mit einem Elrepho RFC-3/24-Gerät gegenüber einem Bariumsulfat-Standard, dessen Remission zu 100 % gesetzt wurde. Die Angabe erfolgt in %-Remission (%-R). Es galten folgende Bedingungen: Flotte 250 ml; Flottenbelastung 1 Gew.-Teil Gewebe/30 Gew.-% Teile Wasser; Dosierung 10 g/l; Wasserhärte 16°d; Mittel von 3 Bestimmungen.

Die Ergebnisse der Versuche sind in Tabelle 1a-c zusammengefaßt. Die Beispiele 1 bis 17 sind erfindungsgemäß, die Beispiele V1 bis V4 dienen zum Vergleich.

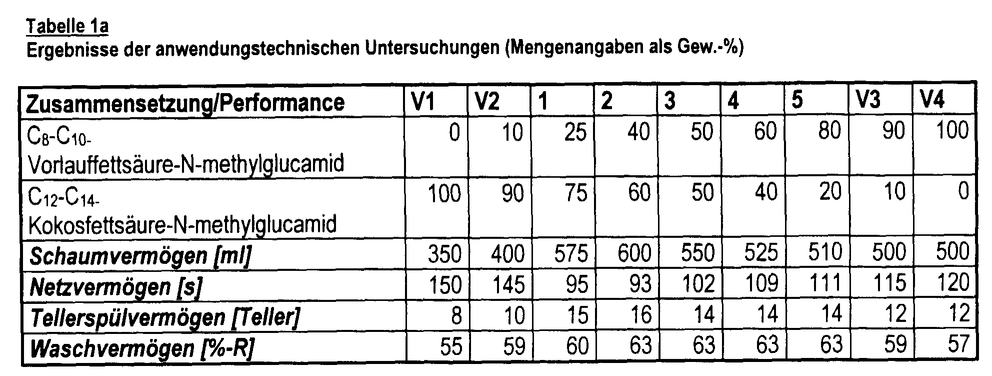

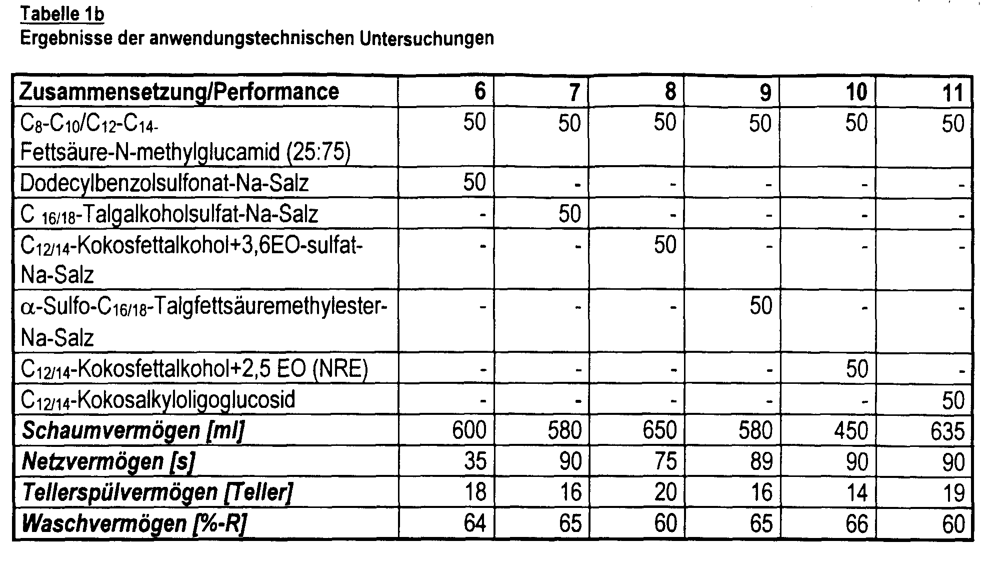

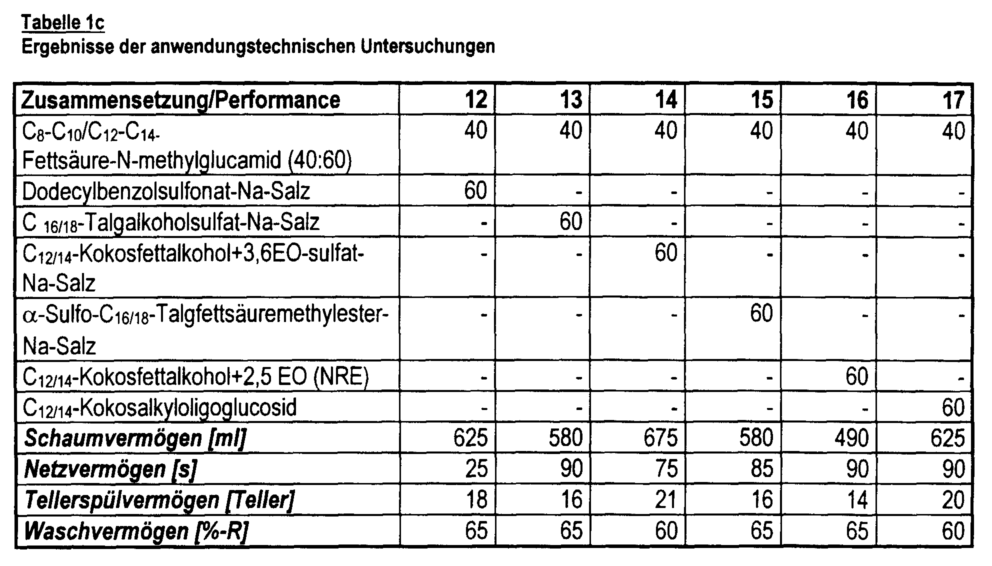

## Patentansprüche

1. Tensidgemische mit verbesserten oberflächenaktiven Eigenschaften, enthaltend
(a) Fettsäure-N-alkylpolyhydroxyalkylamide der Formel **(I)**, 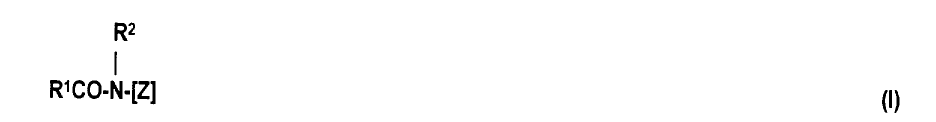 in der R¹CO für einen aliphatischen Acylrest mit 6 bis 10 Kohlenstoffatomen, R² für einen Alkyl- oder Hydroxyalkylrest mit 1 bis 4 Kohlenstoffatomen und [Z] für einen linearen oder verzweigten Polyhydroxyalkylrest mit 3 bis 12 Kohlenstoffatomen und 3 bis 10 Hydroxylgruppen steht, und
(b) Fettsäure-N-alkylpolyhydroxyalkylamide der Formel **(II)**, 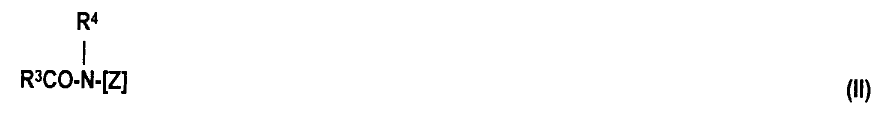 in der R³CO für einen aliphatischen Acylrest mit 12 bis 22 Kohlenstoffatomen, R⁴ für einen Alkyl- oder Hydroxyalkylrest mit 1 bis 4 Kohlenstoffatomen und [Z] für einen linearen oder verzweigten Polyhydroxyalkylrest mit 3 bis 12 Kohlenstoffatomen und 3 bis 10 Hydroxylgruppen steht,
wobei das Gewichtsverhältnis zwischen den Komponenten (a) und (b) 80 : 20 bis 20 : 80 beträgt.

2. Tensidgemische nach Anspruch 1, **dadurch gekennzeichnet**, daß sie als Komponente (a) Fettsäure-N-alkylglucamide der Formel **(I)** enthalten, in der R¹CO für einen gesättigten Acylrest mit 8 bis 10 Kohlenstoffatomen und R² für einen Methylrest steht.

3. Tensidgemische nach den Ansprüchen 1 und 2, **dadurch gekennzeichnet**, daß sie als Komponente (b) Fettsäure-N-alkylglucamide der Formel **(II)** enthalten, in der R³CO für einen gesättigten Acylrest mit 12 bis 18 Kohlenstoffatomen und R⁴ für einen Methylrest steht.

4. Tensidgemische nach den Ansprüchen 1 bis 3, **dadurch gekennzeichnet**, daß sie die Komponenten (a) und (b) im Gewichtsverhältnis 75 : 25 bis 25 : 75 enthalten.

5. Tensidgemische nach den Ansprüchen 1 bis 4, **dadurch gekennzeichnet,** daß sie weitere anionische, nichtionische, kationische, amphotere und oder zwitterionische Tenside enthalten.

6. Tensidgemische nach Anspruch 5, **dadurch gekennzeichnet**, daß sie anionische Tenside enthalten, die ausgewählt sind aus der Gruppe, die von Alkylbenzolsulfonaten, Alkansulfonaten, Olefinsulfonaten, Alkylethersulfonaten, Glycerinethersulfonaten, p-Methylestersulfonaten, Sulfofettsäuren, Alkylsulfaten, Fettalkoholethersulfaten, Glycerinethersulfaten, Hydroxymischethersulfaten, Monoglycerid(ether)sulfaten, Fettsäureamid(ether)sulfaten, Sulfosuccinaten, Sulfotriglyceriden, Amidseifen, Ethercarbonsäuren, Fettsäureisethionaten, Sarcosinaten, Tauriden, Alkyloligoglucosidsulfaten, Alkyl(ether)phosphaten, Acyllactylaten und pflanzlichen oder tierischen Eiweißhydrolysaten bzw. deren Kondensationsprodukten mit Fettsäuren gebildet wird.

7. Tensidgemische nach Anspruch 5, **dadurch gekennzeichnet**, daß sie nichtionische Tenside enthalten, die ausgewählt sind aus der Gruppe, die von Fettalkoholpolyglycolethern, Alkylphenolpolyglycolethem, Fettsäurepolyglycolestern, Fettsäureamidpolygylcolethem, Fettaminpolyglycolethern, alkoxylierten Triglyceriden, Alk(en)yloligoglykosiden, Polyolfettsäureestern, Zuckerestern, Sorbitanestern und Polysorbaten gebildet wird.

8. Tensidgemische nach Anspruch 5, **dadurch gekennzeichnet**, daß sie kationische Tenside enthalten, die ausgewählt sind aus der Gruppe, die von quartären Ammoniumverbindungen und quaternierten Fettsäuretrialkanolaminestern gebildet wird.

9. Tensidgemische nach Anspruch 5, **dadurch gekennzeichnet**, daß sie amphotere bzw. zwitterionische Tenside enthalten, die ausgewählt sind aus der Gruppe, die von Alkylbetainen, Alkylamidobetainen, Aminopropionaten, Aminoglycinaten, Imidazoliniumbetainen und Sulfobetainen gebildet wird.

10. Pulverförmige Universalwaschmittel, enthaltend 10 bis 30 Gew.-% - bezogen auf das Mittel - der Tensidgemische nach Anspruch 1 sowie gegebenenfalls weitere Hilfs- und Zusatzstoffe.

11. Flüssige Universalwaschmittel, enthaltend 10 bis 70 Gew.-% - bezogen auf das Mittel - der Tensidgemische nach Anspruch 1 sowie gegebenenfalls weitere Hilfs- und Zusatzstoffe.

12. Flüssige Feinwaschmittel, enthaltend 10 bis 50 Gew.-% - bezogen auf das Mittel - der Tensidgemische nach Anspruch 1 sowie gegebenenfalls weitere Hilfs- und Zusatzstoffe.

13. Flüssige Reinigungs- und Desinfektionsmittel, enthaltend 10 bis 30 Gew.-% - bezogen auf das Mittel - der Tensidgemische nach Anspruch 1 sowie gegebenenfalls weitere Hilfs- und Zusatzstoffe.

14. Handgeschirrspülmittel, enthaltend 5 bis 25 Gew.-% - bezogen auf das Mittel - der Tensidgemische nach Anspruch 1 sowie gegebenenfalls weitere Hilfs- und Zusatzstoffe.

15. Maschinengeschirrspülmittel, enthaltend 5 bis 25 Gew.-% - bezogen auf das Mittel - der Tensidgemische nach Anspruch 1 sowie gegebenenfalls weitere Hilfs- und Zusatzstoffe.

16. Klarspülmittel, enthaltend 10 bis 50 Gew.-% - bezogen auf die Mittel - der Tensidgemische nach Anspruch 1 sowie gegebenenfalls weitere Hilfs- und Zusatzstoffe.

17. Wäscheweichspülmittel, enthaltend 2 bis 35 Gew.-% - bezogen auf die Mittel - der Tensidgemische nach Anspruch 1 sowie gegebenenfalls weitere Hilfs- und Zusatzstoffe.

18. Haarshampoos, enthaltend 10 bis 30 Gew.-% - bezogen auf das Mittel - der Tensidgemische nach Anspruch 1 sowie gegebenenfalls weitere Hilfs- und Zusatzstoffe.

19. Haarspülungen, enthaltend 10 bis 30 Gew.-% - bezogen auf das Mittel - der Tensidgemische nach Anspruch 1 sowie gegebenenfalls weitere Hilfs- und Zusatzstoffe.

20. Schaumbäder, enthaltend 10 bis 30 Gew.-% - bezogen auf das Mittel - der Tensidgemische nach Anspruch 1 sowie gegebenenfalls weitere Hilfs- und Zusatzstoffe.

21. Syndetseifen, enthaltend 10 bis 50 Gew.-% - bezogen auf das Mittel - der Tensidgemische nach Anspruch 1 sowie gegebenenfalls weitere Hilfs- und Zusatzstoffe.

22. Sammler für die Flotation nichtsulfidischer Erze, enthaltend 10 bis 95 Gew.-% - bezogen auf die Mittel - der Tensidgemische nach Anspruch 1 sowie gegebenenfalls weitere Hilfs- und Zusatzstoffe.

23. Verwendung der Tensidgemische nach Anspruch 1 zur Herstellung von Wasch-, Spül-, Reinigungs- und Flotationshilfsmitteln sowie Produkten zur Haar- und Körperpflege.

## Claims

1. Surfactant mixtures with improved surface-active properties containing
a) fatty acid-N-alkyl polyhydroxyalkylamides corresponding to formula (I): 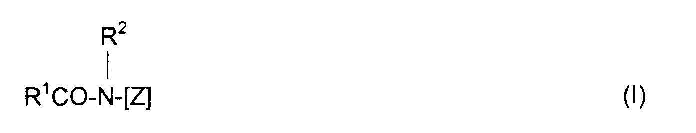 in which R¹CO is an aliphatic acyl radical containing 6 to 10 carbon atoms, R² is an alkyl or hydroxyalkyl radical containing 1 to 4 carbon atoms and [Z] is a linear or branched polyhydroxyalkyl radical containing 3 to 12 carbon atoms and 3 to 10 hydroxyl groups, and
b) fatty acid-N-alkyl polyhydroxyalkylamides corresponding to formula (II): 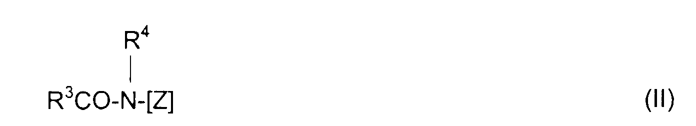 in which R³CO is an aliphatic acyl radical containing 12 to 22 carbon atoms, R⁴ is an alkyl or hydroxyalkyl radical containing 1 to 4 carbon atoms and [Z] is a linear or branched polyhydroxyalkyl radical containing 3 to 12 carbon atoms and 3 to 10 hydroxyl groups,
the ratio by weight between components a) and b) being from 80:20 to 20:80.

2. Surfactant mixtures as claimed in claim 1, characterized in that they contain fatty acid-N-alkyl glucamides corresponding to formula (I), in R¹CO is a saturated acyl radical containing 8 to 10 carbon atoms and R² is a methyl radical, as component a),

3. Surfactant mixtures as claimed in claims 1 and 2, characterized in that they contain fatty acid-N-alkyl glucamides corresponding to formula (II), in which R³CO is a saturated acyl radical containing 12 to 18 carbon atoms and R⁴ is a methyl group, as component b).

4. Surfactant mixtures as claimed in claims 1 to 3, characterized in that they contain components a) and b) in a ratio by weight of 75:25 to 25:75.

5. Surfactant mixtures as claimed in claims 1 to 4, characterized in that they contain other anionic, nonionic, cationic, amphoteric and/or zwitterionic surfactants.

6. Surfactant mixtures as claimed in claim 5, characterized in that they contain anionic surfactants selected from the group consisting of alkyl benzene sulfonates, alkane sulfonates, olefin sulfonates, alkyl ether sulfonates, glycerol ether sulfonates, α-methyl ester sulfonates, sulfofatty acids, alkyl sulfates, fatty alcohol ether sulfates, glycerol ether sulfates, hydroxy mixed ether sulfates, monoglyceride (ether) sulfates, fatty acid amide (ether) sulfates, sulfosuccinates, sulfotriglycerides, amide soaps, ether carboxylic acids, fatty acid isethionates, sarcosinates, taurides, alkyl oligoglucoside sulfates, alkyl (ether) phosphates, acyl lactylates and vegetable or animal protein hydrolyzates or condensation products thereof with fatty acids.

7. Surfactant mixtures as claimed in claim 5, characterized in that they contain nonionic surfactants selected from the group consisting of fatty alcohol polyglycol ethers, alkylphenol polyglycol ethers, fatty acid polyglycol esters, fatty acid amide polyglycol ethers, fatty amine polyglycol ethers, alkoxylated triglycerides, alk(en)yl oligoglycosides, polyol fatty acid esters, sugar esters, sorbitan esters and polysorbates.

8. Surfactant mixtures as claimed in claim 5, characterized in that they contain cationic surfactants selected from the group consisting of quaternary ammonium compounds and quaternized fatty acid trialkanolamine esters.

9. Surfactant mixtures as claimed in claim 5, characterized in that they contain amphoteric or zwitterionic surfactants selected from the group consisting of alkyl betaines, alkyl amidobetaines, aminopropionates, aminoglycinates, imidazolinium betaines and sulfobetaines.

10. Powder-form universal detergents containing 10 to 30% by weight, based on the detergent, of the surfactant mixtures claimed in claim 1 and optionally other auxiliaries and additives.

11. Liquid heavy-duty detergents containing 10 to 70% by weight, based on the detergent, of the surfactant mixtures claimed in claim 1 and optionally other auxiliaries and additives.

12. Liquid light-duty detergents containing 10 to 50% by weight, based on the detergent, of the surfactant mixtures claimed in claim 1 and optionally other auxiliaries and additives.

13. Liquid cleaners and disinfectants containing 10 to 30% by weight, based on the detergent, of the surfactant mixtures claimed in claim 1 and optionally other auxiliaries and additives.

14. Manual dishwashing detergents containing 5 to 25% by weight, based on the detergent, of the surfactant mixtures claimed in claim 1 and optionally other auxiliaries and additives.

15. Machine dishwashing detergents containing 5 to 25% by weight, based on the detergent, of the surfactant mixtures claimed in claim 1 and optionally other auxiliaries and additives.

16. Rinse aids containing 10 to 50% by weight, based on the rinse aid, of the surfactant mixtures claimed in claim 1 and optionally other auxiliaries and additives.

17. Fabric softeners containing 2 to 35% by weight, based on the fabric softener, of the surfactant mixtures claimed in claim 1 and optionally other auxiliaries and additives.

18. Hair shampoos containing 10 to 30% by weight, based on the shampoo, of the surfactant mixtures claimed in claim 1 and optionally other auxiliaries and additives.

19. Hair rinses containing 10 to 30% by weight, based on the hair rinse, of the surfactant mixtures claimed in claim 1 and optionally other auxiliaries and additives.

20. Foam baths containing 10 to 30% by weight, based on the foam bath, of the surfactant mixtures claimed in claim 1 and optionally other auxiliaries and additives.

21. Syndet soaps containing 10 to 50% by weight, based on the syndet soap, of the surfactant mixtures claimed in claim 1 and optionally other auxiliaries and additives.

22. Collectors for the flotation of non-sulfidic ores containing 10 to 95% by weight, based on the collector, of the surfactant mixtures claimed in claim 1 and optionally other auxiliaries and additives.

23. The use of the surfactant mixtures claimed in claim 1 for the production of dishwashing detergents, laundry detergents, cleaning compositions and flotation auxiliaries and hair-care and body-care products.

## Revendications

1. Mélanges d'agents tensioactifs ayant des propriétés détergentes améliorées contenant :
a) des N-alkylpolyhydroxyalkylamides d'acide gras de formule (I) : 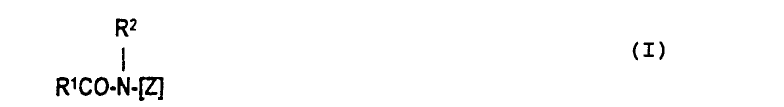 dans laquelle R¹CO représente un radical acyle aliphatique ayant de 6 à 10 atomes de carbone, R² représente un radical alkyle ou hydroxyalkyle ayant de 1 à 4 atomes de carbone et [Z] représente un radical polyhydroxyalkyle linéaire ou ramifié, ayant de 3 à 12 atomes de carbone et de 3 à 10 groupes hydroxy,
b) et des N-alkylpolyhydroxyalkylamides d'acide gras de formule (II) : 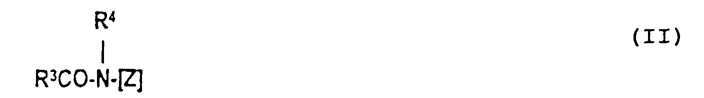 dans laquelle R³CO représente un radical acyle aliphatique ayant de 12 à 22 atomes de carbone, R⁴ un radical alkyle ou hydroxyalkyle ayant de 1 à 4 atomes de carbone, et [Z] un radical polyhydroxyalkyle linéaire ou ramifié, ayant de 3 à 12 atomes de carbone et de 3 à 10 groupes hydroxyles,
pour lesquels le rapport en poids entre les composants a) et b) s'élève à 80:20 à 20:80.

2. Mélanges d'agents tensioactifs selon la revendication 1,
caractérisés en ce qu'
ils contiennent comme composants (a) des N-alkylglucamides d'acide gras de formule (I) dans laquelle R¹CO représente un radical acyle saturé ayant de 8 à 10 atomes de carbone et R² représente un radical méthyle.

3. Mélanges d'agents tensioactifs selon les revendications 1 et 2,
caractérisé en ce qu'
ils contiennent comme composant (b) des N-alkylglucamides d'acide gras de formule (II) dans laquelle R³CO représente un radical acyle saturé ayant de 12 à 18 atomes de carbone et R⁴ représente un radical méthyle.

4. Mélanges d'agents tensioactifs selon les revendications 1 à 3,
caractérisés en ce qu'
ils renferment les composants (a) et (b) dans un rapport en poids allant de 75:25 à 25:75.

5. Mélanges d'agents tensioactifs selon les revendications 1 à 4,
caractérisés en ce qu'
ils renferment d'autres agents tensioactifs anioniques, non ioniques, cationiques, amphotères et/ou zwitterioniques.

6. Mélanges d'agents tensioactifs selon la revendication 5,
caractérisés en ce qu'
ils renferment des agents tensioactifs anioniques choisis dans le groupe qui est formé des alkylbenzènesulfonates, des alkanesulfonates, des oléfinesulfonates, des alkyléthersulfonates, des glycéroléther sulfonates, des µ-esterméthyliquesulfonates, des acides gras sulfonés, des alkylsulfates, des sulfates d'éther d'alcool gras, des sulfates d'éther de glycérol, des sulfates d'éthers mixtes hydroxylés, des sulfates de monoglycéride(éther), des sulfates d'amide d'acide gras (éther), des sulfosuccinates, des sulfotriglycérides, des savons d'amide, des acides éther carboxyliques, des iséthionates d'acide gras, des sarcosinates, des taurides, des alkyloligoglucosides sulfates, des alkyl(éther)phosphates, des acyllactylates, et des hydrolysats d'albumine végétale ou animale ou leurs produits de condensation avec des acides gras.

7. Mélanges d'agents tensioactifs selon la revendication 5,
caractérisés en ce qu'
ils renferment des agents tensioactifs non ioniques qui sont choisis dans le groupe qui est formé des éthers de polyglycol et d'acide gras, des éthers de polyglycol et d'alkylphénol, des esters de polyglycol d'acide gras, des éthers de polyglycol d'amide d'acide gras, des éthers de polyglycol d'amine grasse, des triglycérides alkoxylés, des alk(én)yloligoglycosides, des esters de polyol d'acide gras, des esters de sucre, des esters de sorbitanne et des polysorbates.

8. Mélanges d'agents tensioactifs selon la revendication 5,
caractérisés en ce qu'
ils renferment des agents tensioactifs cationiques qui sont choisis dans le groupe qui est formé par les composés d'ammonium quaternaires et les esters de trialkanolamine d'acide gras quaternisés.

9. Mélanges d'agents tensioactifs selon la revendication 5,
caractérisés en ce qu'
ils renferment des agents tensioactifs amphotères ou zwitterioniques qui sont choisis dans le groupe qui est formé par les alkylbétaïnes, les alkylamidobétaïnes, les amino-propionates, les aminoglycinates, les bétaïnes d'imidazolinium et les sulfobétaïnes.

10. Produit de lavage universel pulvérulent renfermant de 10 à 30 % en poids, rapporté au produit, de mélanges d'agents tensioactifs selon la revendication 1 ainsi qu'éventuellement d'autres adjuvants et additifs.

11. Produit de lavage universel liquide renfermant de 10 à 70 % en poids, rapporté au produit, de mélanges d'agents tensioactifs selon la revendication 1 ainsi qu'éventuellement d'autres adjuvants et additifs.

12. Produit de lavage fin liquide contenant de 10 à 50 % en poids, rapporté au produit, de mélanges d'agents tensioactifs selon la revendication 1 ainsi qu'éventuellement d'autres adjuvants et additifs.

13. Produits de nettoyage et de désinfection liquides renfermant de 10 à 30 % en poids, rapporté au produit, de mélanges d'agents tensioactifs selon la revendication 1, ainsi qu'éventuellement d'autres adjuvants et additifs.

14. Produit de lavage de la vaisselle à la main, renfermant de 5 à 25 % en poids, rapporté au produit, de mélanges d'agents tensioactifs selon la revendication 1, ainsi qu'éventuellement d'autres adjuvants et additifs.

15. Produit de lavage de la vaisselle à la machine renfermant de 5 à 25 % en poids, rapporté au produit, de mélanges d'agents tensioactifs selon la revendication 1, ainsi qu'éventuellement d'autres adjuvants et additifs.

16. Produit de rinçage clair renfermant de 10 à 50 % en poids, rapporté au produit, de mélanges d'agents tensioactifs selon la revendication 1, ainsi qu'éventuellement d'autres adjuvants et additifs.

17. Produit adoucissant pour le textile renfermant de 2 à 35 % en poids, rapporté au produit, de mélanges d'agents tensioactifs selon la revendication 1, ainsi qu'éventuellement d'autres adjuvants et additifs.

18. Shampooings pour les cheveux renfermant de 10 à 30 % en poids, rapporté au produit, de mélanges d'agents tensioactifs selon la revendication 1, ainsi qu'éventuellement d'autres adjuvants et additifs.

19. Rinçages pour les cheveux renfermant de 10 à 30 % en poids, rapporté au produit, de mélanges d'agents tensioactifs selon la revendication 1, ainsi qu'éventuellement d'autres adjuvants et additifs.

20. Bain moussant renfermant de 10 à 30 % en poids, rapporté au produit, de mélanges d'agents tensioactifs selon la revendication 1, ainsi qu'éventuellement d'autres adjuvants et additifs.

21. Savons de détergents synthétiques renfermant de 10 à 50 % en poids, rapporté au produit, de mélanges d'agents tensioactifs selon la revendication 1, ainsi qu'éventuellement d'autres adjuvants et additifs.

22. Produit de recueil pour la flotation de minerais non sulfurés renfermant de 10 à 95 % en poids, rapporté au produit, de mélanges d'agents tensioactifs selon la revendication 1, ainsi qu'éventuellement d'autres adjuvants et additifs.

23. Utilisation des mélanges d'agents tensioactifs selon la revendication 1 pour la préparation d'adjuvants de lavage, de rinçage, de nettoyage, et de flotation ainsi que de produits pour les soins des cheveux et du corps.
